# EUROPEAN PATENT APPLICATION

(11) **EP 2 206 492 A1**
(43) Date of publication of application: **14.07.2010**
(21) Application number: 08847914.2
(22) Date of filing: 05.11.2008
(51) Int. Cl.: A61K 8/60, A61K 8/19, A61K 8/25, A61K 8/92, A61Q 1/04

(54) **OILY COSMETIC AND COSMETIC PRODUCT**

(30) Priority: 05.11.2007 JP 2007287100
(71) Applicant: Shiseido Company, Ltd., Chuo-ku Tokyo 104-8010 (JP)
(72) Inventor: OGURA, Yuuki, Yokohama-shi Kanagawa 224-8558 (JP); MIYAZAKI, Takayuki, Yokohama-shi Kanagawa 224-8558 (JP); SAITO, Yuuko, Yokohama-shi Kanagawa 224-8558 (JP)
(74) Representative: Ulmann, Catherine Claire
(86) International application number: PCT/JP2008/070136
(87) International publication number: WO 2009/060870

(57) **Abstract**

A composition is prepared to comprise (a) 50 to 99% by weight of an oily phase that is a liquid at 25°C, (b) 1 to 7% by weight of dextrin (palmitate/2-ethylhexanoate), and (c) 0.001 to 10% by weight of a coloring material and/or pearlescent agent other than a laminated film powder are contained, followed by making the viscosity thereof at 30°C when measured with a BL viscometer to be 3,000 to 20,000 mPa·s. In addition, (d) silicic acid anhydride and/or hydrophobized silicic acid anhydride is added thereto.

## Description

### OILY COSMETIC AND COSMETIC PRODUCT TECHNICAL FIELD

The present invention relates to an oily cosmetic, and more particularly, to an oily cosmetic and cosmetic product in which a coloring material and/or pearlescent agent are stably dispersed while retaining fluidity.

### BACKGROUND ART

Oily cosmetics constitute a form of cosmetics that are commonly used in eye shadow, eyebrow, lipstick and other cosmetic products, and which demonstrate various functions, textures, colors and the like by changing the types and contents thereof of solid oils, semi-solid oils, liquid oils, coloring pigments, photoluminescent pigments, extender powders and the like incorporated therein. This texture, color and the like are important elements in makeup cosmetics, and various technologies related thereto have been proposed in the past. For example, pearlescent materials using mica as a matrix or coloring materials composed of laminated powders of polyethylene terephthalate, metal and epoxy resin (Patent Documents 1 and 2), and colored metal pigments in which a silicon dioxide layer and a layer containing a coloring substance are formed on the surface of a base pigment composed of aluminum, copper, zinc, iron, gold bronze, silver or an alloy thereof (Patent Document 3) have been developed.
Although there are few problems with dispersion stability over time since dispersed coloring materials and the like are fixed within these oily cosmetics incorporating a pearlescent material or coloring material in the case they are of the solid type, in the case of oily cosmetics having fluidity, even though there may be no problems with dispersibility immediately after production, since coloring materials and the like precipitate over time, there has been a need to develop an oily cosmetic that offers a high level of dispersion stability. In addition, in the case of filling into a transparent container, residual fluid flow within the bottle has conventionally been uneven, thereby making this undesirable in terms of appearance. Consequently, although these oily cosmetics have been filled into opaque containers, if the residual fluid flow thereof was uniform, these oily cosmetics could be filled into transparent containers, thereby expanding the range of product selection. Consequently, there has been a need to develop an oily cosmetic that can be filled into a transparent container and demonstrate uniform residual fluid flow and an appealing appearance. In addition, since brush-type applicators are soft on the skin and enable uniform application, lip gloss contained in a transparent bottle provided with a brush applicator have begun to appear on the market in recent years. However, if conventional solid-type oily cosmetics are used for these applications, there was the problem of fibers on the end of the brush separating when inserting and removing the brush and difficulty in stirring the contents in the top and bottom of the bottle with the soft brush due to the contents of the bottle being excessively hard.

Patent Document 1: Japanese Unexamined Patent Publication (Kokai) No. H11-349446
Patent Document 2: Japanese Unexamined Patent Publication (Kokai) No. H11-349433
Patent Document 3: Japanese Unexamined International Patent Publication (Kohyo) No. 2007-515526

### DISCLOSURE OF THE INVENTION

### Problems to be Solved by the Invention

In order to solve the problems of the prior art as described above, an object of the present invention is to provide an oily cosmetic that inhibits precipitation of coloring materials and pearlescent materials, demonstrates an attractive appearance and has fluidity. In addition, an object of the present invention is to provide an oily cosmetic that can be filled into a transparent bottle and demonstrate uniform residual fluid flow for an attractive appearance, can be easily removed from the bottle until it is completely used up, and can inhibit precipitation of coloring materials and pearlescent materials.

### Means for Solving the Problems

The inventors of the present invention found that by using a specific dextrin fatty acid ester, precipitation of coloring materials and pearlescent materials can be inhibited in an oily cosmetic having fluidity, thereby leading to completion of the present invention.

Namely, the present invention is an oily cosmetic that comprising:
(a) 50 to 99% by weight of an oily phase that is a liquid at 25°C,
(b) 1 to 7% by weight of dextrin (palmitate/2-ethylhexanoate), and
(c) 0.001 to 10% by weight of a coloring material and/or pearlescent agent other than a laminated film powder;
**characterized in that**
viscosity at 30°C when measured with a BL viscometer is 3,000 to 20,000 mPa·s.

### Effects of the Invention

The oily cosmetic of the present invention has fluidity and maintains a uniformly dispersed state without demonstrating precipitation of the coloring material or pearlescent agent.
In addition, the oily cosmetic of the present invention demonstrates an attractive appearance with uniform fluid flow when filled into a transparent container. Moreover, it can be easily removed from the container until it is completely used up and prevents separation of the end of an applicator brush.

### BEST MODE FOR CARRYING OUT THE INVENTION

The following provides an explanation of preferred embodiments of the present invention.
The (a) oily phase that is a liquid at 25°C used in the present invention refers to that in which the entire oily component is in liquid form at 25°C. The oily component may be any oily component provided it is an oily component ordinarily used in cosmetics. The oily component may contain a solid oily component or semi-solid oily component and is required to be in liquid form overall when mixed at 25°C.

Examples of oily components used in the oily cosmetic of the present invention include hydrocarbons, fatty oils, waxes, hydrogenated oils, ester oils, fatty acids, higher alcohols, silicone oils, fluorine-based oils, lanolin derivatives and oily gelling agents regardless of the source, such as an animal oil, vegetable oil or synthetic oil, and regardless of whether in the form of a solid oil, semi-solid oil, liquid oil or volatile oil.

Specific examples of oily components include hydrocarbons such as liquid paraffin, heavy liquid isoparaffin, α-olefin oligomers, squalane, vaseline, polyisobutylene, polybutene, paraffin wax, ceresin wax, microcrystalline wax, polyethylene wax, ethylenepropylene copolymer, Japan wax, montan wax or Fischer-Tropsch wax, oils such as olive oil, castor oil, jojoba oil, mink oil or macadamia nut oil, waxes such as beeswax, carnauba wax, candelilla wax or spermaceti wax, esters such as cetyl isooctanoate, isopropyl myristate, isopropyl palmitate, octyldodecyl myristate, polyglyceryl diisostearate, neopentyglycol dioctanoate or cholesterol fatty acid esters, fatty acids such as stearic acid, lauric acid, myristic acid, behenic acid, isostearic acid or oleic acid, higher alcohols such as stearyl alcohol, cetyl alcohol, lauryl alcohol, oleyl alcohol, isostearyl alcohol or behenyl alcohol, silicones such as lowly polymerized dimethyl polysiloxane, highly polymerized dimethyl polysiloxane, methylphenyl polysiloxane, decamethyl cyclopentasiloxane, octamethyl cyclotetrasiloxane, polyether-modified polysiloxane, polyoxyalkylene- alkylmethylpolysiloxane-methylpolysiloxane copolymer, alkoxy-modified polysiloxane, crosslinked organopolysiloxane or fluorine-modified polysiloxane, fluorine-based oily agents such as perfluorodecane, perfluorooctane or perfluoropolyether, lanolin and lanolin derivatives such as lanolin acetate, lanolin isopropyl fatty acid or lanolin alcohol, and oily gelling agents such as sucrose fatty acid esters, starch fatty acid esters, aluminum 12-hydroxystearate or calcium stearate.

The incorporated amount of the oily phase is preferably 50 to 99% by weight and more preferably 90 to 98% by weight. Incorporation within these ranges is preferable in terms of ease of use and stability.

The (b) dextrin (palmitate/2-ethylhexanoate) used in the present invention refers to that having a dextrin fatty acid ester in the form of a fatty acid portion and both palmitic acid and 2-ethylhexanoic acid, or that having a mixture of dextrin palmitate and dextrin 2-ethylhexanoate. Examples of commercially available products thereof include Rheopearl LL and Rheopearl TT" (Chiba Flour Milling Co., Ltd.).

The incorporated amount of the dextrin (palmitate/2-ethylhexanoate) used in the present invention is 1 to 7% by weight and preferably 1 to 5% by weight. The use of dextrin fatty acid esters other than dextrin (palmitate/ 2-ethylhexanoate) prevents the obtaining of satisfactory performance in terms of dispersion stability and appearance. If the incorporated amount of the dextrin (palmitate/ 2-ethylhexanoate) is less than 1% by weight, dispersion stability becomes poor, while if the incorporated amount exceeds 7% by weight, the resulting product lacks fluidity.

The (c) coloring material and/or pearlescent agent other than a laminated film powder used in the present invention may be any coloring material or pearlescent agent other than a laminated film powder ordinarily used in cosmetics.

Examples of coloring materials include inorganic white pigments (such as titanium dioxide or zinc oxide); inorganic red pigments (such as iron oxide (bengala) or iron titanate); inorganic brown pigments (such as γ-iron oxide); inorganic yellow pigments (such as yellow iron oxide or loess); inorganic black pigments (such as black iron oxide or low-dimensional titanium oxide); inorganic violet pigments (such as manganese violet or cobalt violet); inorganic green pigments (such as chromium oxide, chromium hydroxide or cobalt titanate); inorganic blue pigments (such as ultramarine or Prussian blue); and, metal powders (such as aluminum, gold, silver or copper);
organic pigments such as zirconium, barium or aluminum lake pigments (such as Red No. 2, Red No. 3, Red No. 102, Red No. 104, Red No. 105, Red No. 106, Red No. 201, Red No. 202, Red No. 203, Red No. 204, Red No. 205, Red No. 206, Red No. 207, Red No. 208, Red No. 213, Red No. 214, Red No. 215, Red No. 218, Red No. 219, Red No. 220, Red No. 221, Red No. 223, Red No. 225, Red No. 226, Red No. 227, Red No. 228, Red No. 230, Red No. 231, Red No. 232, Red No. 401, Red No. 404, Red No. 405, Red No. 501, Red No. 502, Red No. 503, Red No. 504, Red No. 505, Red No. 506, Orange No. 201, Orange No. 205, Orange No. 401, Yellow No. 4, Yellow No. 5, Yellow No. 201, Yellow No. 202, Yellow No. 203, Yellow No. 204, Yellow No. 205, Yellow No. 401, Yellow No. 402, Yellow No. 403(1), Yellow No. 404, Yellow No. 405, Yellow No. 406, Yellow No. 407, Blue No. 1, Blue No. 404, Green No. 3, Green No. 201, Green No. 202, Green No. 204 or Violet No. 201); and,
natural pigments (such as β-carotene, cochineal pigments, red cabbage pigments, riboflavin, red ocher, anthraquinone, canthaxanthin or anthraquinone safflower pigments).

In addition, pearlescent agents having mica for the matrix thereof are preferable for the pearlescent agents other than a laminated film powder, examples of which include titanium oxide-coated mica in which the surface thereof is coated with titanium oxide, iron titanium oxide-coated mica, such as red iron oxide-coated titanated mica, in which mica is coated with iron oxide and titanium oxide, and powders in which silica is sandwiched between mica and a titanium oxide coating layer. Examples of powders that have a matrix other than mica include powders in which titanium oxide is coated on silica flakes, powders in which titanium oxide is coated on synthetic mica, and hollow titanium oxide.

Examples of commercially available pearlescent agents include Flamenco Super Pearl, Flamenco Orange, Flamenco Sparkle, Flamenco ultra Sparkle 4500, Cloisonne Orange, Cloisonne Blue, Cloisonne Antique Blue, Cloisonne Cerise Flambe, Cloisonne Sparkle (gold, copper, blue, rouge), Timica Golden Bronze, Timica Nu-Antique Copper, Timica Gold Sparkle, Timica Extra Large Sparkle, Flamenco Satin Red, Flamenco Satin Violet, Flamenco Satin Blue, Flamenco Sparkle (red, gold, green, blue, violet), Duchrome BV (all of the above products are available from Engelhard Corp.), Timilon Super, Timilon Super Silk MP-1005, Timilon Super Sheen MP-1001, Timilon Star Luster MP-115, Colorona Sienna, Colorona Red Gold, Colorona Red Brown, Colorona Bright Gold, Colorona Bordeaux, Colorona Imperial Red, Colorona Sienna Sparkle, Timilon Gold Plus MP-25, Timilon Splendid (for the above products, Merck & Co., Inc.), Prestige Series (ECKART GmbH & Co.), Metashine 1080-RC (B1, G1, R1, S1, Y1) (Nippon Sheet Glass Co., Ltd.), and Prominence Series (Topy Industries, Ltd.).

The incorporated amount of the (c) coloring material and/or pearlescent agent other than a laminated film powder used in the present invention is 0.001 to 10% by weight and preferably 0.1 to 5% by weight.

The oily cosmetic of the present invention has a viscosity of the system of 3,000 to 20,000 mPa·s, and preferably 5,000 to 15,000 mPa·s, when measured with a BL viscometer at 30°C. If the viscosity is less than 3,000 mPa·s, the oily cosmetic lacks dispersibility and stability of the coloring materials, while if the viscosity exceeds 20,000 mPa·s, the oily cosmetic demonstrates hardly any fluidity, thereby preventing it from being an oily cosmetic having fluidity that is an object of the present invention.

In the present invention, an oily cosmetic having favorable stability is obtained regardless of the type of component (c) by further containing (d) silicic acid anhydride and/or hydrophobized silicic acid anhydride.

Fine granular silica having a primary particle diameter of about 5 to 50 nm is preferably used for the (d) silicic acid anhydride or hydrophobized silicic acid anhydride used in the present invention. Hydrophobized silicic acid anhydride is obtained by treating silicic acid anhydride with dimethyldichlorosilane and the like. The component (d) is preferably hydrophobized silicic acid anhydride and more preferably methylpolysiloxane-coated silicic acid anhydride.
Examples of commercially available products of silicic acid anhydride or hydrophobized silicic acid anhydride include Aerosil R972, #200, #380 or RY200 (Nippon Aerosil Co., Ltd.), and HDK (registered trademark) H15 or H20 (Wacker Asahikasei Silicone Co., Ltd.).

The incorporated amount of the (d) silicic acid anhydride and/or hydrophobized silicic acid anhydride used in the present invention is preferably 0.01 to 3% by weight and more preferably 0.5 to 2% by weight. If the incorporated amount of the (d) silicic acid anhydride and/or hydrophobized silicic acid anhydride is less than 0.01% by weight, the desired effects are not obtained, while if the incorporated amount exceeds 3% by weight, the viscosity of the system ends up exceeding 20,000 mPa·s, thereby impairing fluidity.

Various types of components for imparting various effects other than the aforementioned essential components can be suitably incorporated as necessary in the oily cosmetic of the present invention to a degree that does not impair the effects of the present invention, examples of which include powders other than those previously described, surfactants, aqueous components, film forming agents, ultraviolet absorbers, moisturizers, fading preventive agents, antioxidants, antifoaming agents, aesthetic components, antiseptics and fragrances.

Aqueous components used for the purpose of imparting moisturizing effects can be water or any aqueous component that dissolves in water, examples of which include, in addition to water, alcohols such as ethyl alcohol or isopropyl alcohol, glycols such as propylene glycol, 1,3-butylene glycol, dipropylene glycol or polyethylene glycol, glycerols such as glycerin, diglycerin or polyglycerin, and plant extracts such as aloe vera, witch hazel, hamamelis, cucumber, lemon, lavender or rose extract.

Examples of ultraviolet absorbers include benzophenone-based, PABA-based (paraaminobenzoic acid-based), cinnamic acid-based, salicylic acid-based, 4-tert-butyl-4'- methoxydibenzoylmethane and oxybenzone-based ultraviolet absorbers, while examples of moisturizers include proteins, mucopolysaccharides, collagen, elastin and keratin. Examples of antioxidants include α-tocopherol and ascorbic acid, examples of aesthetic components include vitamins, antiphlogistic agents and natural herbs, while examples of antiseptics include paraoxybenzoic acid esters and phenoxyethanol.

Examples of the oily cosmetic of the present invention include cosmetics used for skin care, makeup and hair care, with makeup cosmetics being particularly preferable. Examples of makeup cosmetics include eye color (eye shadow), eye brow, lip cream, lip gloss, foundation, blush, mascara and enamel, with lip gloss being particularly preferable.

According to the present invention, a cosmetic product is provided that comprises filling the aforementioned oily cosmetic into a transparent container. Since the uniformly and stably dispersed oily cosmetic is filled into a transparent container without the occurrence of precipitation of the coloring material or pearlescent agent, residual adherence of the oily cosmetic to the walls of the container is uniform, appearance is appealing, and that appealing appearance is maintained without changing over time, thereby enhancing product appeal.
There are no particular limitations on the container into which the oily cosmetic of the present invention is filled provided it is a typical container that allows a cosmetic to be filled therein. Examples of such containers include bottles, pen-shaped containers, tubes, metal dish containers and containers provided with an applicator. Examples of applicators include brushes and flocked tips, while examples of container materials include glass, polyethylene (PE), polypropylene/ethylene vinyl alcohol resin (PE,PP/EVOH)laminate), polypropylene/ethylene vinyl alcohol resin (PP,PP/EVOH)laminate), polyethylene terephthalate (PET) and acrylonitrile-butadiene- styrene copolymer synthetic resin (ABS).

### Examples

Although the following provides a more detailed description of the present invention through examples thereof, the present invention is not limited to these examples. In the examples, incorporated amounts are indicated in % by weight unless specifically indicated otherwise.

### Test Examples 1-1 to 1-11 (Examples Examining Types and Incorporated Amounts of Component (b))

Lip gloss having the compositions shown in the following Table 1 were prepared in accordance with ordinary methods followed by evaluations of viscosity, fluidity and appearance immediately after production as well as stability both immediately after production and up to four weeks later using the methods described below. The results are also shown in Table 1.

### (1) Viscosity Immediately After Production

Viscosity was measured using a BL viscometer manufactured by Shibaura Systems Co., Ltd. immediately after production. Measurement conditions consisted of a temperature of 30°C and the use of a No. 3 rotor at 12 rpm. However, a No. 4 rotor at 12 rpm were used for measuring viscosity immediately after production for Test Examples 1-10 and 1-11 since viscosity exceeded the measurement limit under the conditions described above.

### (2) Fluidity

Fluidity was evaluated immediately after production.

### (3) Appearance

Measurement conditions: Allowing to stand undisturbed after allowing the contents to spread throughout the container followed by observing one hour later.

### (Evaluation Criteria)

Excellent (EX): Extremely good fluidity and no residual fluid flow (uniformly flows out).
Good (GD): No residual fluid flow observed (uniformly flows out)
Acceptable (OK): Some residual fluid flow observed, but appearance not impaired
Unacceptable (NG): Residual fluid flow observed that impairs appearance

### (4) Stability (Immediately After Production)

Stability immediately after production was evaluated according to the following criteria.

### (Evaluation Criteria)

Excellent (EX): No precipitation of coloring material or pearlescent agent
Good (GD): Some precipitation of coloring material or pearlescent agent and transparent layer (oily portion) of 2 mm or less
Acceptable (OK): Some precipitation of coloring material or pearlescent agent and transparent layer (oily portion) of 5 mm or less
Unacceptable (NG): Precipitation of coloring material or pearlescent agent and transparent layer of 5 mm or more

### (5) Stability (After 1, 2 or 4 Weeks)

Stability was evaluated according to the following criteria after allowing the lip gloss to stand undisturbed for 1, 2 or 4 weeks at 50°C in an inverted tube (beneath the cap), upright tube (above the cap), vertical bottle (above the cap), horizontal bottle and a screw tube.

### (Evaluation Criteria)

Excellent (EX): No precipitation of coloring material or pearlescent agent
Good (GD): Some precipitation of coloring material or pearlescent agent and transparent layer (oily portion) of 2 mm or less
Acceptable (OK): Some precipitation of coloring material or pearlescent agent and transparent layer (oily portion) of 5 mm or less (Note: "Cracking" indicates observation of a crack of about 5 mm in the side of the contents)
Unacceptable (NG): Precipitation of coloring material or pearlescent agent and transparent layer of 5 mm or more

### (6) Ease of Removal with Applicator

The ease with which contents can be removed with the applicator until completion of use was evaluated according to the following criteria.

### (Evaluation Criteria)

Excellent (EX): No dripping or no residual contents observed at completion of use
Good (GD): No residual contents observed at completion of use, but some dripping of contents Acceptable (OK): Contents remaining in top and bottom of bottle at completion of use Unacceptable (NG): Contents remaining on sides, top and bottom of bottle at completion of use

### (7) Separation of Brush Tip

The brush was removed from the container and the lip gloss was applied over the entirety of the lips followed by replacing the cap. This procedure was repeated twice a day for 2 weeks. Separation of the brush tip was then evaluated according to the following criteria following 2 weeks of continuous use in this manner.

### (Evaluation Criteria)

Excellent (EX): No separation of brush tip observed
Good (GD): Separation of 1 to 3 brush fibers observed
Acceptable (OK): Separation of 4 to 9 brush fibers observed
Unacceptable (NG): Entire brush twisted and separation of 10 or more fibers observed

**[Table 1]**

| Test Example | 1-1 | 1-2 | 1-3 | 1-4 | 1-5 | 1-6 | 1-7 | 1-8 | 1-9 | 1-10 | 1-11 |
|---|---|---|---|---|---|---|---|---|---|---|---|
| Dextrin palmitate^{*1} | 0.3 | 0.5 | 0.8 | 1 | -- | -- | -- | -- | -- | -- | -- |
| Dextrin (palmitate/2-ethylhexanoate)^{*2} | -- | -- | -- | -- | 0.5 | 1 | 1.5 | 2 | 5 | 7 | 10 |
| Polyglyceryl tristearate^{*3} | 5 | 5 | 5 | 5 | 5 | 5 | 5 | 5 | 5 | 5 | 5 |
| Macadamia nut oil^{*4} | 0.1 | 0.1 | 0.1 | 0.1 | 0.1 | 0.1 | 0.1 | 0.1 | 0.1 | 0.1 | 0.1 |
| Diisostearyl malate^{*5} | 43 | 42.8 | 42.5 | 42.3 | 42.8 | 42.3 | 41.8 | 41.3 | 38.3 | 36.3 | 33.3 |
| Polybutene^{*6} | 50 | 50 | 50 | 50 | 50 | 50 | 50 | 50 | 50 | 50 | 50 |
| Dipropylene glycol | 1 | 1 | 1 | 1 | 1 | 1 | 1 | 1 | 1 | 1 | 1 |
| Coloring material (Red No. 202 and black iron oxide:oil = 9:1) | 0.1 | 0.1 | 0.1 | 0.1 | 0.1 | 0.1 | 0.1 | 0.1 | 0.1 | 0.1 | 0.1 |
| Titanium oxide-coated synthetic fluorophlogopite^{*7} | 0.5 | 0.5 | 0.5 | 0.5 | 0.5 | 0.5 | 0.5 | 0.5 | 0.5 | 0.5 | 0.5 |
| Total (%) | 100 | 100 | 100 | 100 | 100 | 100 | 100 | 100 | 100 | 100 | 100 |
| Immediately after production | | | | | | | | | | | |
| Viscosity | 5460 | 7020 | 9000 | 9680 | 4580 | 5120 | 5800 | 6920 | 17800 | 17900 | 27100 |
| Fluidity | Yes | Yes | Yes | Yes | Yes | Yes | Yes | Yes | Yes | Little | None (flowed in one night) |
| Appearance | OK | OK | OK | OK | EX | EX | EX | EX | GD | GD | OK |
| Stability (immediately after production) | EX | EX | EX | EX | NG | EX | EX | EX | EX | EX | EX |
| Stability (4 weeks) | | | | | | | | | | | |
| 50°C, inverted tube (below cap) | -- | EX | EX | EX | NG | OK | GD | GD | -- | -- | -- |
| 50°C, upright tube (above cap) | -- | OK | OK | OK | NG | OK | GD | GD | -- | -- | -- |
| 50°C, screw tube | -- | EX | EX | EX | NG | EX | EX | EX | -- | -- | -- |
| Ease of removal with applicator | EX | EX | EX | EX | EX | EX | EX | EX | OK | OK | NG |
| Separation of brush tip | GD | GD | GD | OK | GD | GD | EX | EX | NG | NG | NG |

- *1:: Rheopearl KL (Chiba Flour Milling Co., Ltd.)
- *2:: Rheopearl TT (Chiba Flour Milling Co., Ltd.)
- *3:: Cosmol 43V
- *4:: TZ Oil
- *5:: Cosmol 222
- *6:: Deodorized polybutene
- *7:: Prominence YF

As can be understood from Table 1, lip gloss having an attractive appearance was unable to be obtained in products using dextrin palmitate. In addition, the use of dextrin (palmitate/2-ethylhexanoate) allowed the obtaining of a stable base having a viscosity of 5,000 mPa·s or more (1% by weight or more) and free of nearly all problems.
In Test Example 1-5, the high content of a highly viscous oil component contributed to viscosity of the oily portion, and although the viscosity of the system was 4,580 mPa·s as a result thereof, since the content of dextrin (palmitate/2-ethylhexanoate) was low at 0.5% by weight, the effect of this component of increasing viscosity was low and stability was not maintained. In addition, there was hardly any fluidity observed when the content of dextrin (palmitate/2-ethylhexanoate) was 10% by weight.

### Text Examples 2-1 to 2-5 (Examples of Changing Type of Coloring Material)

Lip gloss having the compositions shown in the following Table 2 was prepared in accordance with ordinary methods followed by evaluation of stability four weeks later using the previously described method. The results are also shown in Table 2.

**[Table 2]**

| Test Example | 2-1 | 2-2 | 2-3 | 2-4 | 2-5 |
|---|---|---|---|---|---|
| Dextrin (palmitate/2-ethylhexanoate)^{*2} | 1.5 | 1.5 | 1.5 | 1.5 | 1.5 |
| Polyglyceryl tristearate^{*3} | 5 | 5 | 5 | 5 | 5 |
| Macadamia nut oil^{*4} | 0.1 | 0.1 | 0.1 | 0.1 | 0.1 |
| Diisostearyl malate^{*5} | 41.65 | 41.65 | 41.65 | 41.65 | 41.65 |
| Polybutene^{*6} | 50 | 50 | 50 | 50 | 50 |
| Hydrogenated soybean phospholipid^{*8} | 0.1 | 0.1 | 0.1 | 0.1 | 0.1 |
| Butylmethoxydibenzoylmethane^{*9} | 0.1 | 0.1 | 0.1 | 0.1 | 0.1 |
| Calcium stearate | 0.05 | 0.05 | 0.05 | 0.05 | 0.05 |
| Dipropylene glycol | 0.5 | 0.5 | 0.5 | 0.5 | 0.5 |
| Yellow No. 4 | 1 | -- | -- | -- | -- |
| Yellow No. 5 | -- | 1 | -- | -- | -- |
| Red No. 202 | -- | -- | 1 | -- | -- |
| Red No. 201 | -- | -- | -- | 1 | -- |
| Blue No. 1 | -- | -- | -- | -- | 1 |
| Total (%) | 100 | 100 | 100 | 100 | 100 |
| Stability (4 weeks) | | | | | |
| 50°C, inverted tube (below cap) | EX | EX | EX | EX | EX |
| 50°C, upright tube (above cap) | EX | EX | EX | EX | EX |
| 50°C, screw tube | EX | EX | EX | EX | EX |

- *8:: Basis LP-20H
- *9:: Pearlsol 1789

As can be understood from Table 2, the lip gloss was confirmed to be free of problems with stability even when using various types of coloring materials.

### Test Examples 3-1 to 3-8 (Examples of Changing Type of Pearlescent Agent)

Lip gloss having the compositions shown in the following Table 3 was prepared in accordance with ordinary methods followed by evaluation of stability immediately after production, stability after allowing to stand undisturbed for 1 week at 50°C in a screw tube, and stability after allowing to stand undisturbed for 1 week at room temperature in a screw tube using the previously described methods. The results are also shown in Table 3.

**[Table 3]**

| Test Example | 3-1 | 3-2 | 3-3 | 3-4 | 3-5 | 3-6 | 3-7 | 3-8 |
|---|---|---|---|---|---|---|---|---|
| Dextrin (palmitate/2-ethylhexanoate)^{*2} | 1.5 | 1.5 | 1.5 | 1.5 | 1.5 | 1.5 | 1.5 | 1.5 |
| Polyglyceryl tristearate^{*3} | 5 | 5 | 5 | 5 | 5 | 5 | 5 | 5 |
| Macadamia nut oil^{*4} | 0.1 | 0.1 | 0.1 | 0.1 | 0.1 | 0.1 | 0.1 | 0.1 |
| Diisostearyl malate^{*5} | 39.65 | 39.65 | 39.65 | 39.65 | 39.65 | 39.65 | 39.65 | 39.65 |
| Polybutene^{*6} | 50 | 50 | 50 | 50 | 50 | 50 | 50 | 50 |
| Hydrogenated soybean phospholipid^{*8} | 0.1 | 0.1 | 0.1 | 0.1 | 0.1 | 0.1 | 0.1 | 0.1 |
| Butylmethoxydibenzoylmethane^{*9} | 0.1 | 0.1 | 0.1 | 0.1 | 0.1 | 0.1 | 0.1 | 0.1 |
| Calcium stearate | 0.05 | 0.05 | 0.05 | 0.05 | 0.05 | 0.05 | 0.05 | 0.05 |
| Dipropylene glycol | 0.5 | 0.5 | 0.5 | 0.5 | 0.5 | 0.5 | 0.5 | 0.5 |
| Dimethicone-treated titanated mica^{*10} | 3 | -- | -- | -- | -- | -- | -- | -- |
| Titanated mica^{*11} | -- | 3 | -- | -- | -- | -- | -- | -- |
| Iron oxide-treated titanated mica^{*12} | -- | -- | 3 | -- | -- | -- | -- | -- |
| Red iron oxide-coated mica^{*13} | -- | -- | -- | 3 | -- | -- | -- | -- |
| Titanium oxide-coated synthetic phlogopite^{*14} | -- | -- | -- | -- | 3 | -- | -- | -- |
| Titanium oxide-coated borosilicate glass^{*15} | -- | -- | -- | -- | -- | 3 | -- | -- |
| Titanium oxide-coated borosilicate glass^{*16} | -- | -- | -- | -- | -- | -- | 3 | -- |
| Polyethylene terephthalate-polymethyl | -- | -- | -- | -- | -- | -- | -- | 3 |
| methacrylate laminated film powder^{*17} | | | | | | | | |
| Total (%) | 100 | 100 | 100 | 100 | 100 | 100 | 100 | 100 |
| Stability immediately after production | EX | EX | EX | EX | EX | EX | EX | NG |
| Stability (1 week) | | | | | | | | |
| 50°C, 1 week, screw tube | EX | EX | EX | OK | GD | OK | EX | NG |
| RT, 1 week, screw tube | EX | EX | EX | EX | GD | GD | EX | NG |

- *10:: Flamenco Super Pearl (Marl Corp.)
- *11:: SA Flamenco Super Pearl (Miyoshi Kasei, Inc.)
- *12:: Timilon MP-29 (Merck & Co., Inc.)
- *13:: Colorona Glitter Bronze (Merck & Co., Inc.)
- *14:: Prominence SF (Topy Industries, Ltd.)
- *15:: Reflex Silver (Engelhard Corp.)
- *16:: Metashine MC 1020RS (Nippon Sheet Glass Co., Ltd.)
- *17:: Aurora Flake R100 (Kakuhachi Co., Ltd.)

As can be understood from Table 3, precipitation preventive effects were confirmed for all of the pearlescent agents excluding the laminated film powder in the form of Aurora Flake, and the lip gloss was confirmed to be free of problems with stability.

### Test Example 4

Lip gloss having the composition shown in the following Table 4 was prepared in accordance with ordinary methods followed by evaluation of viscosity immediately after production, stability immediately after production, stability after allowing to stand undisturbed at 50°C for 1 week in a screw tube, and stability after allowing to stand at room temperature (25°C) for 1 week in a screw tube using the previously described methods. The results are also shown in Table 4.

**[Table 4]**

| Test Example | 4 |
|---|---|
| Dextrin (palmitate/2-ethylhexanoate)^{*2} | 1.5 |
| Methylpolysiloxane-coated silicic acid anhydride^{*18} | 0.5 |
| Polyglyceryl tristearate^{*3} | 5 |
| Macadamia nut oil^{*4} | 0.1 |
| Diisostearyl malate^{*5} | 41.8 |
| Polybutene^{*6} | 30 |
| Liquid paraffin | -- |
| Pentaerythrityl 2-ethylhexanoate | 20 |
| Hydrogenated soybean phospholipid^{*8} | -- |
| Butylmethoxydibenzoylmethane^{*9} | -- |
| Calcium stearate | -- |
| Dipropylene glycol | 0.5 |
| Coloring material (Red No. 202 and black iron oxide:oil = 9:1) | 0.1 |
| Titanium oxide-coated synthetic fluorophlogopite^{*7} | 0.5 |
| Total (%) | 100 |
| Viscosity (no. 3 rotor, 6 rpm, BL viscometer) | 3300 |
| Stability (immediately after production) | EX |
| Stability (1 week) | |
| 50°C, 1 week, screw tube | EX |
| RT, 1 week, screw tube | EX |

### *18: Aerosil RY200S

As can be understood from Table 4, precipitation preventive effects were confirmed at a viscosity of 3300 mPa·s, and the lip gloss was confirmed to be free of problems with stability.

### Test Examples 5-1 to 5-3 (Examples of Changing Type of Silicic Acid Anhydride)

Lip gloss having the compositions shown in the following Table 5 was prepared in accordance with ordinary methods followed by evaluation of stability immediately after production and stability after allowing to stand undisturbed at 50°C for 2 weeks in bottle using the previously described methods. The results are also shown in Table 5.

**[Table 5]**

| Test Example | 5-1 | 5-2 | 5-3 |
|---|---|---|---|
| Dextrin (palmitate/2-ethylhexanoate) | 2 | 2 | 2 |
| Polybutene | 40 | 40 | 40 |
| Diisostearyl malate | 38.83 | 38.83 | 38.83 |
| Pentaerythrityl 2-ethylhexanoate^{*19} | 15.5 | 15.5 | 15.5 |
| Dimethylsilyl silicic acid anhydride*²⁰ | 0.5 | -- | -- |
| Silicic acid anhydride^{*21} | -- | 0.5 | -- |
| Methylpolysiloxane-coated silicic acid anhydrde^{*22} | -- | -- | 0.5 |
| Polyethylene glycol | 0.1 | 0.1 | 0.1 |
| Hyaluronic acid-containing powder | 0.1 | 0.1 | 0.1 |
| Nylon | 0.05 | 0.05 | 0.05 |
| Tocopherol | 0.01 | 0.01 | 0.01 |
| Tocopherol acetate | 0.01 | 0.01 | 0.01 |
| Dipropylene glycol | 0.5 | 0.5 | 0.5 |
| Butylmethoxybenzoylmethane | 0.1 | 0.1 | 0.1 |
| Hydrogenated soybean phospholipid | 0.1 | 0.1 | 0.1 |
| Calcium stearate | 0.05 | 0.05 | 0.05 |
| Coloring matter^{*23} | 0.5 | 0.5 | 0.5 |
| Titanium oxide-coated borosilicate glass^{*24} | 1.6 | 1.6 | 1.6 |
| Dimethicone | 0.05 | 0.05 | 0.05 |
| Total | 100 | 100 | 100 |
| Viscosity immediately after production | 4600 | 4600 | 4800 |
| Fluidity | Yes | Yes | Yes |
| Stability (immediately after production) | EX | EX | EX |
| Stability (50°C, 2 weeks, bottle, vertical) | EX | EX | EX |
| Stability (50°C, 2 weeks, bottle, horizontal) | GD | GD | EX |
| Ease of removal with applicator | EX | EX | EX |
| Separation of brush tip | EX | EX | EX |

| | | | |
|---|---|---|---|
| * Cosmol 168M semi-solid oil ** Blended Coloring Material Titanium oxide Tetrahydrotetramethyl cyclotetrasiloxane Iron oxide Methicone Tetradecene Silica Barium sulfate TCL444 Yellow No. 4 TCL243 Yellow No. 5 TCL282 Red No. 202 TCL444 Red No. 201 TLC450 Blue No. 1 TCL741 Red No. 218 Dye F Orange No. 201 Dye D *** Pearlescent Agent Titanium oxide-coated borosilicate glass: Metashine Series Red iron oxide-coated silicic acid anhydride: Sirona Indian Summer Titanium oxide-Silicic acid anhydride composite-coated mica: Timilon Splendid Series Iron oxide-coated titanated mica: Prism Tone Powder, Diophase FE Silver, Cloisonne Nu-Antique Flambe Titanated mica: Timilon Super Series, Timilon MP-45 Red iron oxide-coated titanated mica: Timica Brilliant Gold *19: RA-PE-408 (Nippon Fine Chemical Co., Ltd.) *20: Aerosil R972 *21: Aerosil #200 *22: Aerosil RY200S *23: Blended coloring material (iron oxide, methicone, tetradecene, barium sulfate, Red No. 202, Red No. 218) *24: Metashine 1080RY | | | |

As can be understood from Table 5, lip gloss incorporating various types of silicic acid anhydride at 0.5% was confirmed to be free of problems with stability at viscosities of 4,000 to 5,000 mPa·s. In particular, incorporation of methylpolysiloxane-coated silicic acid anhydride having a high degree of hydrophobicity resulted in uniform dispersion of the pearlescent agent, and this was the most effective in preventing precipitation. In addition, at incorporated amounts of silicic acid anhydride of 2% by weight or more, cracking phenomenon was observed and the lip gloss no longer demonstrated fluidity.

### Examples 6-1 to 6-4 (Examples of Changing

### Incorporated Amount of Methylpolysiloxane-coated Silicic Acid Anhydride)

Lip gloss having the compositions shown in the following Table 6 was prepared in accordance with ordinary methods followed by evaluation of stability immediately after production and stability after allowing to stand undisturbed at 50°C for 4 weeks in bottle using the previously described methods. The results are also shown in Table 6.

**[Table 6]**

| Test Example | 6-1 | 6-2 | 6-3 | 6-4 |
|---|---|---|---|---|
| Dextrin (palmitate/2-ethylhexanoate) | 2 | 2 | 2 | 2 |
| Polybutene | 40 | 40 | 40 | 40 |
| Diisostearyl malate | 38.98 | 38.98 | 38.98 | 38.98 |
| Pentaerythrityl 2-ethylhexanoate | 15.5 | 15.5 | 15.5 | 15.5 |
| Methylpolysiloxane-coated silicic acid anhydride | | 0 1 | 2 | 3 |
| Polyethylene glycol | 0.1 | 0.1 | 0.1 | 0.1 |
| Hyaluronic acid-containing powder | 0.1 | 0.1 | 0.1 | 0.1 |
| Tocopherol | 0.01 | 0.01 | 0.01 | 0.01 |
| Tocopherol acetate | 0.01 | 0.01 | 0.01 | 0.01 |
| Dipropylene glycol | 0.5 | 0.5 | 0.5 | 0.5 |
| Butylmethoxybenzoylmethane | 0.1 | 0.1 | 0.1 | 0.1 |
| Hydrogenated soybean phospholipid | 0.1 | 0.1 | 0.1 | 0.1 |
| Calcium stearate | 0.05 | 0.05 | 0.05 | 0.05 |
| Coloring matter^{*25} | 1.5 | 1.5 | 1.5 | 1.5 |
| Titanated mica^{*26} | 1 | 1 | 1 | 1 |
| Dimethicone | 0.05 | 0.05 | 0.05 | 0.05 |
| Total | 100 | 101 | 102 | 103 |
| Viscosity (no. 3 rotor, BL viscometer, 30°C) | 4200 | 6500 | 14000 | 32000 |
| Fluidity | Yes | Yes | Some | No |
| Stability (immediately after production) | EX | EX | EX | EX |
| Stability (50°C, 4 weeks, bottle, vertical) | EX | EX | OK (cracks) | OK (cracks) |
| Stability (50°C, 4 weeks, bottle, | EX | EX | OK | OK |
| horizontal) | | | (cracks) | (cracks) |
| Ease of removal with applicator | GD | EX | OK | NG |
| Separation of brush tip | EX | EX | OK | NG |

| | | | | |
|---|---|---|---|---|
| *25: Blended coloring material (titanium oxide, tetrahydrotetramethyl cyclotetrasiloxane, iron oxide, methicone, tetradecene, barium sulfate, Yellow No. 4, Red No. 202) *26: Flamenco Gold | | | | |

As can be understood from Table 6, lip gloss incorporating methylpolysiloxane-coated silicic acid anhydride was confirmed to demonstrate precipitation preventive effects and be free of problems with stability at viscosities of 4,000 to 6,500 mPa·s.

### Example 1 (Lip Gloss)

| | |
|---|---|
| Dextrin (palmitate/2-ethylhexanoate) (Rheopearl TT) | 1.5 wt% |
| Heavy liquid isoparaffin | 50 |
| Diisostearyl malate | 40 |
| Polyglyceryl-2 triisostearate | 5 |
| Iron oxide-Red No. 201 blended coloring material | 0.5 |
| Titanium oxide-coated synthetic fluorophlogopite (Prominence SF) | 3 |

### (Production Method)

Dextrin (palmitate/2-ethylhexanoate) was mixed with diisostearyl malate at room temperature followed by heating to 100°C, incorporating the other components and again heating to 100°C, followed by stirring and mixing, degassing and filling into a transparent container at 80°C.

### Example 2 (Lip Gloss)

| | |
|---|---|
| Dextrin (palmitate/2-ethylhexanoate) (Rheopearl TT) | 1.5 wt% |
| Heavy liquid isoparaffin | 50 |
| Diisostearyl malate | 40 |
| Polyglyceryl-2 triisostearate | 5 |
| Iron oxide-Red No. 201 blended coloring material | 0.5 |
| Polyethylene terephthalate-polymethyl methacrylate laminated film powder (Aurora Flake R100) | 3 |
| Hydrophobized silicic acid anhydride | 0.5 |

### (Aerosil R972)

### (Production Method)

Dextrin (palmitate/2-ethylhexanoate) and hydrophobized silicic acid anhydride were mixed with diisostearyl malate at room temperature followed by heating to 100°C, incorporating the other components and again heating to 100°C, followed by stirring and mixing, degassing and filling at 80°C.

## Claims

1. An oily cosmetic, comprising:
(a) 50 to 99% by weight of an oily phase that is a liquid at 25°C,
(b) 1 to 7% by weight of dextrin (palmitate/2-ethylhexanoate), and
(c) 0.001 to 10% by weight of a coloring material and/or pearlescent agent other than a laminated film powder; **characterized in that**
viscosity at 30°C when measured with a BL viscometer is 3,000 to 20,000 mPa·s.

2. The oily cosmetic according to claim 1, **characterized by** further comprising:
(d) silicic acid anhydride and/or hydrophobized silicic acid anhydride.

3. A cosmetic product further **characterized in that** the oily cosmetic according to claim 1 or claim 2 is filled into a transparent container.
